# EUROPÄISCHE PATENTSCHRIFT

(11) **EP 3 145 581 B1**
(45) Veröffentlichungstag und Bekanntmachung des Hinweises auf die Patenterteilung: **03.07.2019**
(21) Anmeldenummer: 14732093.1
(22) Anmeldetag: 23.05.2014
(51) Int. Cl.: A61N 1/20

(54) **THERAPEUTISCH ANWENDBARE MEHRKANAL- GLEICHSTROMABGABEVORRICHTUNG**
THERAPEUTICALLY APPLICABLE MULTICHANNEL DIRECT CURRENT DELIVERY DEVICE
DISPOSITIF MULTICANAUX DE FOURNITURE DE COURANT CONTINU UTILISABLE À DES FINS THÉRAPEUTIQUES

(43) Veröffentlichungstag der Anmeldung: 29.03.2017
(73) Patentinhaber: Molsberger, Albrecht, 40489 Düsseldorf (DE)
(72) Erfinder: Molsberger, Albrecht, 40489 Düsseldorf (DE)
(74) Vertreter: König Szynka Tilmann von Renesse Patentanwälte Partnerschaft mbB
(86) Internationale Anmeldenummer: PCT/EP2014/060742
(87) Internationale Veröffentlichungsnummer: WO 2015/176778

(56) Entgegenhaltungen:
- WO-A1-93/23112
- WO-A1-02/096511
- WO-A1-2013/175021
- US-A- 5 697 909

## Beschreibung

Die vorliegende Erfindung betrifft eine Vorrichtung zur Abgabe von Gleichstrom. Die erfindungsgemäße Gleichstromabgabevorrichtung ist zur therapeutischen oder kosmetischen Behandlung des menschlichen oder tierischen Körpers einsetzbar. Die vorliegende Erfindung richtet sich außerdem auf die Gleichstromabgabevorrichtung zur spezifischen Anwendung in bestimmten Verfahren zur kosmetischen Behandlung des menschlichen oder tierischen Körpers. Die erfindungsgemäße Gleichstromabgabevorrichtung eignet sich insbesondere zur Behandlung von Entzündungen und/oder Schmerzen. Schließlich betrifft die Erfindung auch ein Verfahren zum Herstellen der Gleichstromabgabevorrichtung.
Viele medizinisch oder kosmetisch relevante Beeinträchtigungen des menschlichen oder tierischen Körpers sind lokal bedingt. Eine medizinisch relevante Beeinträchtigung liegt bei Krankheiten oder Störungen der Funktion des Körpers vor. In diesen Fällen ist grundsätzlich eine therapeutische Behandlung indiziert. Im Fall von Reizzuständen, worunter im Rahmen der vorliegenden Erfindung relativ geringfügige Beeinträchtigungen oder Beschwerden des menschlichen oder tierischen Körpers verstanden werden, die keine Krankheit oder Funktionsstörung darstellen und die nicht therapiewürdig sind, ist oftmals zumindest eine kosmetische, nicht-therapeutische Behandlung sinnvoll.

Die vorliegende Offenbarung betrifft sowohl die nicht-therapeutische kosmetische Behandlung von durch (vorwiegend lokale) Reizzustände verursachten kosmetischen Beeinträchtigungen als auch die Schaffung von neuen Therapiemöglichkeiten für (vorwiegend lokale) medizinisch relevante Beeinträchtigungen des menschlichen oder tierischen Körpers.

Der Begriff der Therapie umfasst hierbei auch die Prophylaxe. "Lokal" bedeutet, dass am oder im Körper ein bestimmtes Areal des Reizzustandes oder der medizinisch relevanten Beeinträchtigung feststellbar ist. Dieses Areal ist vorzugsweise lokal umschrieben und bevorzugt exakt identifizierbar. Das Areal (z.B. Reizareal, Entzündungsareal, Schmerzareal) geht mit bestimmten Symptomen einher und löst die Beeinträchtigung bzw. die Beschwerden aus oder ist damit assoziiert.

Beeinträchtigungen des menschlichen oder tierischen Körpers, die von lokalen Entzündungs- bzw. Schmerzzuständen herrühren, sind beispielsweise aseptische Entzündungen - oft bedingt durch lokale Überlastungen (Distorsionen, Ansatztendinosen) oder auch neuropathische Schmerzen. Im orthopädischen Bereich treten lokale Entzündungs- und Schmerzzustände insbesondere auf im Zusammenhang mit Gewebsverletzungen, insbesondere von Muskeln (z.B. Muskeltraumata), Nerven, Haut oder Halteapparat, Gefäßverletzungen oder auch im Zusammenhang mit Nervenentzündung, Entzündungen der Sehnen oder Knochen oder Narbenbildung. Oft besteht dabei ein lokal umschriebenes Areal, in dem die Beschwerden feststellbar sind.

Erfüllt eine Beeinträchtigung das Kriterium einer Erkrankung oder Störung der Funktion des Körpers, so ist es in der Regel angezeigt, sie therapeutisch zu behandeln. Beispiele für eine lediglich kosmetisch relevante Beeinträchtigung sind dagegen Faltenbildung, etwa durch erhöhten Muskeltonus bedingt, Fehl- und Schonhaltungen oder lediglich kosmetisch störende Hautveränderungen wie etwa Rötungen.

Verfahren und Mittel zur therapeutischen oder kosmetischen Behandlung von medizinisch oder kosmetisch relevanten Beeinträchtigungen und Beschwerden sind an sich bekannt. Auch wenn z.B. Physiotherapie in manchen Fällen Linderung verschaffen kann, werden hierfür doch in der Regel bestimmte pharmazeutische Wirkstoffe eingesetzt, in erster Linie Cortison, nichtsteroidale Antirheumatika, Analgetika und verwandte Stoffe. Diese haben neben der gewünschten (lokalen) Wirkung meist auch unerwünschte lokale Nebenwirkungen (bei lokalen Gaben von Cortison etwa aseptische Entzündungen) und/oder systemische Nebenwirkungen und können den Stoffwechsel und Hormonhaushalt nachteilig beeinflussen. In vielen Fällen können die herkömmlichen Therapieverfahren einen chronischen Krankheitsverlauf nicht abwenden. Es ist daher sinnvoll, Alternativen zum Einsatz (exogener) Wirkstoffe in Betracht zu ziehen.

Es sind auch Therapieoptionen bekannt, die verstärkten Gebrauch von den intrinsischen Heilungskapazitäten des menschlichen oder tierischen Körpers machen. Eine weit verbreitete Technik ist die auf die traditionelle chinesische Medizin (TCM) zurückgehende Akupunktur und deren Abwandlungen.

Akupunktur ist für die Behandlung bestimmter Beschwerden, etwa bei chronischem Schmerz (z.B. Kopfschmerz, Migräne) allgemein als effektiv und risikoarm anerkannt. So zahlen etwa alle deutschen gesetzlichen Krankenkassen seit dem 1. Januar 2007 insbesondere auf Grundlage der Ergebnisse großer prospektiver und randomisierter Studien (GERAC, German Acupuncture Trials) Akupunkturbehandlungen bei chronischen Kreuzschmerz und chronischem Knieschmerz bei Gonarthrose. Private Krankenversicherungen bezahlen Akupunkturleistungen zur Schmerzbehandlung und nach Einzelfallentscheidung meist auch für weitere Indikationen. Die "Cochrane-Reviews" von 2009 bezeichnen Akupunktur als "eine wertvolle nicht pharmakologische Therapiemöglichkeit bei Patienten mit häufigem episodischem Spannungskopfschmerz" und stellen fest, dass die "Akupunktur bei Migräne mindestens so wirksam, möglicherweise auch wirksamer, als eine medikamentöse prophylaktische Therapie ist, und dies bei geringeren unerwünschten Wirkungen".

Es zeigte sich in den GERAC-Studien, dass kein signifikanter Unterschied bestand zwischen einer Akupunktur an Punkten, die den Vorgaben der TCM folgt und einer Akupunktur an anderen Punkten (sogenannte "Scheinakupunktur"). Positive therapeutische oder kosmetische Effekte sind bei der Anwendung auf lokale Entzündungs- und Schmerzzustände für beide Formen der Akupunktur nachgewiesen. Heutzutage sind auch Akupunkturformen geläufig, die sich nicht am traditionellen theoretischen Hintergrund der TCM orientieren.

Endogene physiologische elektrische Felder sind in der Biologie bekannt. Solche Felder liegen im Bereich von 70 mV/mm (Nervenwachstum bei Hühnern), 140 mV/mm (Wundheilung bei Ratten), 600 mV/mm (Augenlinse von Wirbeltieren) bis 1500 mV/mm (Entwicklung des Neuronalrohrs beim Lurch Axolotl). Je nach Innenwiderstand des betreffenden biologischen Gewebes ergeben sich hierbei Ströme von 10-200 µA. Endogene elektrische Felder bauen sich für eine Zeit von Stunden bis Wochen beispielsweise im Wundbereich, im Bereich des aktiven Zellwachstums sowie bei der Zellmigration auf und scheinen für die Regulation des Zellverhaltens essenziell zu sein.

Der Einsatz von exogenen elektrischen Feldern in der Medizin und Kosmetik ist dem Grunde nach bekannt. Eingesetzt werden hierbei regelmäßig starke und/oder zeitlich veränderliche Felder, wobei die zeitliche Veränderlichkeit etwa durch Wechselspannung oder kurze Gleichspannungsimpulse bewirkt wird. Diese bislang therapeutisch eingesetzten starken elektrischen Felder werden beispielsweise durch hohe Spannungen und regelmäßig durch starke Ströme erzeugt. In diesem Zusammenhang werden Wechselstrom- und Impulsstromgeräte verwendet, um elektrolytischen Effekten an den eingesetzten Elektroden und insbesondere am Körpergewebe entgegenzuwirken.

Bekannt ist etwa die transkutane elektrische Nervenstimulation (TENS). Hierbei werden niederfrequente (1-100 Hz) biphasische Wechselstromimpulse zur Schmerzlinderung eingesetzt, primär zur kurzfristigen "Elektroanalgesie". Die Spannung beträgt bis zu 70 V bei etwa 250 µs Impulsdauer, die Stromstärke bis zu etwa 90 mA. Die Wirkung beruht in erster Linie auf einer Steigerung der zentralen Ausschüttung von Endorphinen. Es ist unklar, ob darüber hinaus auch lokale und längerfristige Effekte im betroffenen Gewebe erzielt werden.

Bekannt ist auch die Elektroakupunktur. Ihr Wirkmechanismus zielt auf die Ausschüttung zentraler schmerzlindernder Substanzen, insbesondere von Enkephalinen, Endorphinen und Dynorphinen.

Wie im Dokument US 2004/0111128 A1 beschrieben, verwendet auch die Elektroakupunktur Wechselströme. Bei der Elektroakupunktur wird ein niederfrequenter Reizstrom gesetzt (Springer Lexikon der Medizin), hierbei ist die Frequenz des elektrischen Signals fest oder variabel (2-10.000 Hz). Eingesetzt werden hierbei wie bei TENS verhältnismäßig starke Ströme, die zwischen 2 und 15 mA liegen. Diese können in dieser Intensität nur gepulst appliziert werden mit einer Impulsdauer von ca. 0,3 - 0,6 ms. Um bei diesen hohen Strömen elektrolytische Effekte am Übergang zwischen Elektrode und biologischem Gewebe zu vermeiden, wird die Polung gewechselt (Wechselstrom). Dementsprechend umfassen die elektrischen Parameter bei der Elektroakupunktur in jedem Fall Frequenz und Intensität (siehe eine unter der URL http://www.icmart.org/index.php?id=198,0,0,1,0,0 abrufbare und von einem Kongress des International Council on Medical Acupuncture and Related Techniques stammende Zusammenfassung über Parameter der Elektroakupunktur).

Zusammenfassend sind die bekannten Geräte zur TENS oder Elektroakupunktur schaltungstechnisch aufwendig, sie arbeiten mit hohen Strömen, kurzen Impulsen und Wechselstrom bestimmter Frequenzen. Ungeachtet dessen ist die Stimulationsdosis ist oft nicht kontrollierbar. Diese Mittel und Verfahren des Stands der Technik zielen auf eine Schmerzlinderung, die auf einer zentralen analgetischen Wirkung beruht. Auf eine lokale Wirkung (etwa eine entzündungshemmende oder regenerationsfördernde) stellen sie nicht ab.

In der Tumortherapie ist eine Gleichstrom-Galvanotherapie mit hohen Stromstärken von 60-80 mA bei einer Spannung von 6-35 V bekannt. Bei dieser Therapie soll es zu einer Zerstörung des Tumorgewebes kommen, beispielsweise durch Nekrotisierung. Eine Zerstörung von Gewebe ist hier also kein zu vermeidender unerwünschter Effekt, sondern im Gegenteil ausdrücklich erwünscht. Das Verfahren macht sich die erhöhte Leitfähigkeit von Tumorgewebe im Vergleich zu gesundem Gewebe zu Nutze, so dass sich der Stromfluss selektiv im Tumorgewebe konzentrieren und dort den Zerfall des Tumors durch elektrolytische und nekrotisierende Effekte bewirken soll.

Gleichstrom wird auch zum transkutanen Transport ionisierbarer Medikamente (lontophorese) eingesetzt. Zum Einsatz kommen Spannungen von etwa 36-60 V und Ströme von etwa 10-30 mA. Um hier lokale Gewebsschädigungen zu vermeiden und eine hohe Dosis an Wirkstoffen elektrophoretisch transportieren zu können, werden großflächige Hautelektroden auf die Haut gesetzt.

Ferner ist eine Anwendung von breitflächigen feuchten Zellstoffelektroden auf der Kopfhaut zur Stimulation des Zentralnervensystems bekannt, zum Beispiel bei Tinnitus (transkranielle Gleichstromstimulation, tDCS). Hierbei wird eine Stromstärke bis 1 mA und eine Spannung von 8-25 V bei konstantem und pulsierendem Strom eingesetzt. Es ist bekannt, dass die entsprechenden durch schwachen Gleichstrom erzeugten elektrischen Felder das Gefäßwachstum fördern, unter anderem über die Ausschüttung von VEGF und ihren Einfluss auf Endothelzellen. Sie führen zu einer Bewegung und Neuanordnung von Zellmembranrezeptoren, erhöhen die Teilungsrate bestimmter Zellen und beschleunigen die Zellwanderung von Epithelzellen. Diese Zellwanderung erfolgt von der Anode (vom positiven Pol) weg und zur Kathode (zum negativen Pol) hin. Im Tierexperiment gibt es Hinweise, dass die periphere Nervenregeneration nach Rückenmarkstrauma beschleunigt werden kann, wobei die Axone der Nervenzellen zur Kathode hin wachsen, die über etwa drei Wochen kopfwärts liegen muss. Klinische Studien am Menschen weisen auf eine Beschleunigung der Wundheilung durch elektrische Felder hin.

Aus der DE 10 2012 010 262 ist eine Gleichstromabgabevorrichtung bekannt, die ein Mittel zum Konstanthalten der Stromstärke bei der Abgabe des Gleichstroms umfasst. Es finden sich darin jedoch keine Angaben, welche Ströme im Einzelnen konstant gehalten werden müssen und insbesondere keine Hinweise auf ein Konstanthalten der individuellen Ströme einzelner Nadeln. Dokument WO-A-2013/175021 offenbart den nächstliegenden Stand der Technik.

Die vorstehenden Ausführungen stellen ebenso wie eine im vorliegenden Text enthaltene Beschreibung beispielhafter Ausführungsformen keinen Verzicht auf bestimmte Ausführungsformen oder Merkmale dar.

Der vorliegenden Erfindung liegt das technische Problem zu Grunde, neuartige Mittel und Verfahren bereitzustellen, mit denen lokale Beeinträchtigungen des menschlichen oder tierischen Körpers, insbesondere solche, die durch Entzündungen und/oder Schmerzen verursacht werden, gelindert oder beseitigt werden können.

Vorzugsweise erlauben die erfindungsgemäßen Mittel und Verfahren eine effektivere, sicherere, reproduzierbarere und/oder nebenwirkungsärmere Anwendung und/oder haben eine schneller eintretende und/oder längerfristige Wirkung als die Mittel und Verfahren des Stands der Technik. Vorzugsweise erlauben die erfindungsgemäßen Mittel und Verfahren die Behandlung von lokalen Beeinträchtigungen, die mit den Mitteln des Stands der Technik nicht angemessen oder überhaupt nicht zu behandeln sind.

Das technische Problem wird gemäß einem ersten Aspekt der vorliegenden Erfindung gelöst durch eine Gleichstromabgabevorrichtung umfassend eine Gleichstromquelle oder eine Einrichtung zum Anschließen an eine Gleichstromquelle und eine erste Elektrode sowie eine zweite Elektrode zum Verbinden mit der Gleichstromquelle, wobei die erste Elektrode als Mehrzahl von Nadeln ausgebildet ist und die zweite Elektrode als flächige Elektrode (vorzugsweise Klebeelektrode), als Nadel oder Mehrzahl unmittelbar miteinander elektrisch leitend verbundener Nadeln ausgebildet ist, dadurch gekennzeichnet, dass die Gleichstromabgabevorrichtung ein oder mehrere Mittel zum jeweiligen Konstanthalten der Stromstärke bei der individuellen Abgabe von Gleichstrom durch Nadeln der ersten Elektrode (insbesondere im Fall der Veränderung eines an einer Nadel der ersten Elektrode anliegenden Widerstands) umfasst, wobei das Konstanthalten vorzugsweise alle Nadeln der ersten Elektrode betrifft.

Die Nadeln der ersten Elektrode sind nicht unmittelbar miteinander elektrisch leitend verbunden. Bevorzugt ist auch eine erfindungsgemäße Gleichstromabgabevorrichtung, bei der die zweite Elektrode als flächige Elektrode (Klebeelektrode) ausgebildet ist.

Unter einer "Klebeelektrode" wird vorliegend eine flächige Elektrode verstanden, die auf die (gegebenenfalls enthaarte) Hautoberfläche aufbringbar und dort fixierbar ist (gegebenenfalls unter Verwendung eines die Leitfähigkeit vermittelnden oder verbessernden Zusatzmaterials wie Elektrodengel oder Elektrodenpaste), vorzugsweise durch Aufkleben.

Unter einer "unmittelbaren" elektrisch leitenden Verbindung wird eine elektrisch leitende Verbindung durch ein einfaches Kabel verstanden oder eine anderweitige elektrisch leitende Verbindung ohne zwischengeschaltete elektrische Bauteile. Ausgeschlossen ist beispielsweise eine elektrisch leitende Verbindung, die allein darauf beruht, dass zwischen den beiden verbundenen Elementen Bauteile eines Schaltkreises liegen wie etwa Widerstände.

Eine flächige Elektrode wird für die Zwecke der vorliegenden Anmeldung zuweilen auch als "Pad" bezeichnet.

Gemäß einem zweiten Aspekt betrifft die vorliegende Offenbarung auch die erfindungsgemäße Gleichstromabgabevorrichtung zur Anwendung bei der Behandlung von Entzündungen und/oder Schmerzen, insbesondere der Muskeln, Nerven, Sehnen oder Knochen.

Anders ausgedrückt ist Gegenstand dieses Aspekts der vorliegenden Offenbarung auch die Verwendung der erfindungsgemäßen Gleichstromabgabevorrichtung zur Behandlung von Entzündungen und/oder Schmerzen, insbesondere der Muskeln, Nerven, Sehnen oder Knochen bzw. die Verwendung der erfindungsgemäßen Gleichstromabgabevorrichtung zur Herstellung einer therapeutischen Vorrichtung zur Behandlung von Entzündungen und/oder Schmerzen, insbesondere der Muskeln, Nerven, Sehnen oder Knochen. Dieser Aspekt der vorliegenden Offenbarung betrifft auch ein Verfahren zur Behandlung von Entzündungen und/oder Schmerzen, insbesondere der Muskeln, Nerven, Sehnen oder Knochen eines Patienten, der einer solchen Behandlung bedarf, umfassend das Einwirkenlassen der erfindungsgemäßen Gleichstromabgabevorrichtung auf den Körper des Patienten.

Das der vorliegenden Erfindung zu Grunde liegende technische Problem wird gemäß einem dritten Aspekt auch gelöst durch ein Kit zum Herstellen einer erfindungsgemäßen Gleichstromabgabevorrichtung (vorzugsweise wie oben beschrieben), welches umfasst: eine Mehrzahl von Nadeln zur Verwendung als erste Elektrode, eine flächige Elektrode (z.B. Klebeelektrode), eine Nadel oder eine Mehrzahl von Nadeln zur Verwendung als zweite Elektrode, ein oder mehrere Mittel zum jeweiligen Konstanthalten der Stromstärke bei der individuellen Abgabe von Gleichstrom durch mit einer Gleichstromquelle verbundene (oder: durch die mit einer Gleichstromquelle verbundenen) Nadeln der ersten Elektrode (insbesondere im Fall der Veränderung eines an den Nadeln der ersten Elektrode jeweils anliegenden Widerstands), und optional Mittel zum unmittelbaren elektrisch leitenden Verbinden einer Mehrzahl von Nadeln.

Der Begriff "umfassend" beinhaltet auch die Bedeutung "bestehend aus" und hat in bevorzugten Ausführungsformen letztere Bedeutung, außer, wo sich aus dem Kontext zwingend etwas anderes ergibt. Entsprechendes gilt für Begriffsvarianten wie etwa "umfassen" und "bestehen aus".

Unter Gleichstrom wird vorliegend ein elektrischer Strom verstanden, dessen Richtung sich nicht ändert und dessen zeitgemittelte Stromstärke sich unter gleichbleibenden Rahmenbedingungen im Wesentlichen nicht ändert. Vorzugsweise ist der Gleichstrom ein "reiner" Gleichstrom, dessen Stromstärke sich unter gleichbleibenden Rahmenbedingungen im Wesentlichen oder überhaupt nicht ändert. Es sind allerdings auch gewisse zeitliche Schwankungen möglich, insbesondere ein "pulsierender" Gleichstrom, bei dem die Stromstärke periodisch um einen bestimmten Mittelwert pendelt, ohne dass sich dabei allerdings die Stromrichtung ändert. Der Gleichstrom ist also vorzugsweise ein um einen zuvor (vorzugsweise konstant) eingestellten Wert undulierender Gleichstrom, Die Undulation erfolgt vorzugsweise mit einer Frequenz zwischen 0,001 und 10 Hz, insbesondere zwischen 0,01 und 1 Hz, beispielsweise 0,1 Hz. Vorzugsweise ist sie rechteck-, sägezahn- und insbesondere sinusförmig. Vorzugsweise beträgt dabei die Auslenkung des Gleichstroms 50% des zuvor eingestellten Werts (d.h. die Werte bewegen sich zwischen 150% und 50% des zuvor eingestellten Werts), insbesondere 40%, 30%, 20%, 15%, 10%, 7,5%, 5%, 2,5% oder 1%.

Die vorliegende Erfindung beruht auf der Entdeckung, dass schwacher Gleichstrom die erwähnten medizinisch oder kosmetisch relevanten Beeinträchtigungen und Beschwerden bessern kann, wenn er über eine Elektrode in einem lokalen elektrischen Gleichspannungsfeld auf den Körper wirkt. Die Wirkungen treten bereits ein, wenn der Gleichstrom sehr schwach ist. Eine besonders gut reproduzierbare Wirkung ist erzielbar, wenn der Gleichstrom konstant ist.

Eine der Erkenntnisse, die der vorliegenden Erfindung zu Grunde liegen ist es, dass die Wirkung dann besonders vorteilhaft ist (z.B. hinsichtlich der Reproduzierbarkeit der Wirkung, der Stärke der Wirkung, der Schnelligkeit des Wirkungseintritts, der Dauer des Anhaltens der Wirkung und/oder der Behandelbarkeit bestimmter Beeinträchtigungen oder Beschwerden), wenn nicht nur insgesamt die Stromstärke des Gleichstroms konstant gehalten wird, sondern die jeweilige Stromstärke bei der individuellen Abgabe von Gleichstrom durch Nadeln der ersten Elektrode konstant gehalten wird. Anders ausgedrückt geben die jeweiligen Nadeln der ersten Elektrode eine konstante Stromstärke ab (vorzugsweise jede einzelne der Nadeln), und nicht nur die erste Elektrode insgesamt. Indem auf diese Weise ein oder mehrere Mittel zum jeweiligen Konstanthalten der Stromstärke des von (den) individuellen Nadeln der ersten Elektrode abgegebenen Gleichstroms vorgesehen sind, entsteht ein Mehrkanalgerät. Jeder einzelne Kanal, d.h. die Stromstärke der einzelnen Nadeln der ersten Elektrode kann somit konstant gehalten werden. Die von einer individuellen Nadel der ersten Elektrode abgegebene Stromstärke wird nachfolgend als "Einzelstromstärke" bezeichnet. Die erfindungsgemäße Gleichstromabgabevorrichtung ist also mit anderen Worten dadurch gekennzeichnet, dass sie ein oder mehrere Mittel zum jeweiligen Konstanthalten der Einzelstromstärken umfasst.

Je nach Anwendung erzielen nadelförmige oder auch flächige Elektroden besonders gute Effekte (z.B. zwei nadelförmige Elektroden oder auch eine nadelförmige in Kombination mit einer flächigen Elektrode, wobei eine nadelförmige Elektrode eine Mehrzahl von Nadeln umfasst und die gegebenenfalls vorhandene flächige Elektrode optional eine Mehrzahl von flächigen Gebilden umfasst). Das erfindungsgemäße angewandte elektrische Feld liegt in der Größenordnung endogener und physiologischer elektrischer Felder.

Die Stromstärke bestimmt die Stärke des elektrischen Felds im Gewebe. Eine konstante Stromstärke (im Gegensatz zu beispielsweise der Einstellung einer konstanten Spannung) ist insoweit vorteilhaft, als etwaige Schwankungen des Widerstands keine Schwankungen der Stromstärke und insbesondere keine Stromspitzen hervorrufen können. Auch interindividuelle Unterschiede des Widerstands können (im Gegensatz zur Einstellung einer konstanten Spannung) nicht zu unterschiedlichen Stromstärken führen.

Die erfindungsgemäße Gleichstromabgabevorrichtung (wie oben beschrieben) umfasst ein oder mehrere Mittel zum jeweiligen Konstanthalten der Stromstärke bei der individuellen Abgabe des Gleichstroms durch den Nadeln der ersten Elektrode (insbesondere im Fall der Veränderung des Widerstands, der an einer oder mehreren der Nadeln der ersten Elektrode (jeweils) anliegt). Vorzugsweise umfasst die Gleichstromabgabevorrichtung ein solches Mittel für jede der Nadeln der ersten Elektrode. Diese(s) Mittel ist/sind dazu ausgebildet, bei der Abgabe des Gleichstroms die Stromstärke insbesondere dann konstant zu halten, wenn sich der an einer oder mehreren Nadeln der ersten Elektrode (jeweils) anliegende Widerstand ändert. Ohne ein solches Mittel besteht häufig die Situation, dass sich der elektrische Widerstand des Körpergewebes (etwa der Haut) während der Behandlung ändert und die Stromstärke dann schwankt. Es ist eine Erkenntnis der vorliegenden Erfindung, dass unter Verwendung des oder der oben beschriebenen Mittel zum Konstanthalten das Ergebnis Beeinträchtigungen oder Beschwerden behandelbar sind, die ansonsten nicht oder nicht adäquat behandelbar sind. Außerdem wurde gefunden, dass ohne solche Mittel zum Konstanthalten der Stromstärke aufgrund eines individuell verschiedenen Widerstands zwischen Haut und Gewebe, der meist im Bereich von 1-40 kΩ (typischerweise 1-10 kΩ) liegt, bei verschiedenen Individuen unterschiedliche Stromstärken beobachtbar sind und dadurch die Behandlungserfolge in gewissem Rahmen variieren. Unter Verwendung des/der Mittel(s) zum Konstanthalten wird ein gleichbleibender Behandlungserfolg unabhängig vom individuell verschiedenen Widerstand zwischen Haut und Gewebe erzielt.

Durch die erfindungsgemäße Gleichstromabgabevorrichtung wird eine Besserung oder Beseitigung von kosmetisch oder medizinisch relevanten Beeinträchtigungen und Beschwerden des Körpers ermöglicht. Diese sind vorzugsweise Entzündungen und/oder Schmerzen und insbesondere lokal bedingt. Erfindungsgemäß kann die Verwendung exogener pharmazeutischer Wirkstoffe bzw. Medikamente reduziert oder vollständig vermieden werden. Dadurch treten die unerwünschten Nebenwirkungen solcher Stoffe in verringerten Maß oder gar nicht auf. Die Beeinträchtigungen und Beschwerden werden bei Einsatz der erfindungsgemäßen Gleichstromabgabevorrichtung dauerhaft oder zumindest langfristig gelindert oder beseitigt oder ihnen vorgebeugt. Bei Wiederholung der Anwendung kann die Wirkung oftmals bis zur dauerhaften Freiheit von den Beeinträchtigungen gesteigert werden.

Der Einsatz der erfindungsgemäßen Gleichstromabgabevorrichtung ist risikoarm, effektiv und arm an oder frei von Nebenwirkungen. Die Wirkung tritt schnell und voraussagbar ein. Die abgegebene Stromdosis ist exakt kontrollierbar. Vorteilhaft ist zudem, dass erfindungsgemäß auch eine Regeneration von durch chronische Entzündungen oder degenerative Prozesse geschädigtem Gewebe erlaubt wird. Die Wirksamkeit gegen Entzündungen und Schmerzen ist erfindungsgemäß erheblich besser als bei der Elektroakupunktur entsprechend dem Stand der Technik.

Die erfindungsgemäße Gleichstromabgabevorrichtung hat bei Anwendung am menschlichen oder tierischen Körper insbesondere eine antiphlogistische und analgetische Wirkung, die beispielsweise bei der Behandlung von (insbesondere lokalen) Entzündungen und Schmerzen, vor allem der Muskeln, Nerven, Sehnen oder Knochen vorteilhaft ist. Behandelbar sind etwa aseptische Entzündungen, Nervenschmerzen (z.B. neuropathischer Schmerz), Kopfschmerz und orthopädische Indikationen, wie etwa Schmerzen an der Brustwirbelsäule oder Schulter, Rückenschmerzen oder Sehnenschmerzen (etwa Tennisarm). Die Entzündungen/Schmerzen können beispielsweise im Zusammenhang stehen mit Gewebsverletzungen (z.B. Muskeln, Nerven, Haut oder Halteapparat, Gefäße), Nervenentzündung, Entzündungen der Sehnen oder Knochen und Narbenbildung.

Die erfindungsgemäße Gleichstromabgabevorrichtung erlaubt ein Behandlungskonzept, das auf der Akupunktur aufbaut. Es kann separat angewendet oder in den üblichen Kontext einer Akupunkturbehandlung eingebunden werden. Ein solches Konzept kann die Akupunktur fortentwickeln und so die entsprechende therapeutische oder kosmetische Behandlung von Beschwerden bzw. Beeinträchtigungen verbessern.

Erfindungsgemäß können mit der erfindungsgemäßen Gleichstromabgabevorrichtung menschliche oder tierische Körper von Patienten. Der Begriff "Patient" ist nicht einschränkend auf eine therapeutische Behandlung zu verstehen, sondern deckt auch eine kosmetische Behandlung ab. Bevorzugte Patienten sind Säugetiere wie Pferde, Hunde, Katzen oder Kamele und insbesondere Menschen.

Bei einem typischen Behandlungsablauf wird zunächst der schmerzende/entzündete Bereich lokalisiert. Beispielsweise werden eine oder mehrere (Metall-)Nadeln dort eingestochen. Die Spitze(n) der Nadel(n) kann/können auf oder außerhalb von Akupunkturpunkten liegen. Die Nadeln werden als erste Elektrode mit einem Pol, vorzugsweise dem negativen Pol der Gleichstromquelle verbunden. Der andere Pol wird mit der zweiten Elektrode verbunden, die vorzugsweise eine Oberflächen-Klebeelektrode in einem anderen Bereich des Körpers ist. Eine solche Oberflächenelektrode ("Pad") wird vorzugsweise über große Muskelgruppen oder Fettschichten gelegt, damit nicht einzelne Nerven durch die Oberflächenelektrode gereizt werden. Zur Behandlung wird ein Strom angelegt, der an jeder einzelnen der Nadeln der ersten Elektrode konstant gehalten wird. Typischerweise lässt der Schmerz bzw. die Entzündung etwa 2 h nach der Behandlung nach, was allerdings je nach Gewebe auch später sein kann. Beispielsweise stellt man bei der Behandlung von Nerven eine schnellere Wirkung fest als bei der Behandlung von Periost. In der Regel tritt eine Wirkung innerhalb von 24 h nach der Behandlung auf.

Das Konstanthalten der Stromstärke ist vorzugsweise ein automatisiertes Konstanthalten. Die erfindungsgemäße Gleichstromabgabevorrichtung enthält demnach vorzugsweise ein oder mehrere automatisierte Mittel zum jeweiligen Konstanthalten der Stromstärke bei der individuellen Abgabe von Gleichstrom durch Nadeln der ersten Elektrode (insbesondere im Fall der Veränderung eines an einer Nadel der ersten Elektrode anliegenden Widerstands), vorzugsweise für jede der Nadeln der ersten Elektrode.

Der elektrische Widerstand R bei der Behandlung wird in erster Linie vom Kontakt der Elektroden mit der Haut und gegebenenfalls vom direkten Umfeld der Nadel(n) bestimmt. Häufig verändert sich der Widerstand im Laufe der Behandlung. Um dennoch eine gleichbleibende Stromstärke I zu gewährleisten, bestehen unter anderem die Möglichkeiten, die Kontaktfläche zwischen Elektroden und Körpergewebe zu verändern, beispielsweise durch Verändern des Anpressdrucks der zweiten Elektrode, oder einen internen Widerstand der erfindungsgemäßen Gleichstromabgabevorrichtung zu verändern.

Vorzugsweise allerdings wird eine Konstanz der Stromstärke I durch entsprechende Veränderung der Spannung U gewährleistet, die jeweils an den einzelnen Nadeln der ersten Elektrode anliegt.

Vorzugsweise ist in der erfindungsgemäßen Gleichstromabgabevorrichtung die jeweilige Stromstärke bei der individuellen Abgabe von Gleichstrom durch (die) Nadeln der ersten Elektrode individuell einstellbar und insbesondere individuell regelbar.

Bevorzugte Mittel zum Konstanthalten der Stromstärke in der erfindungsgemäßen Gleichstromabgabevorrichtung sind automatisiert und als Regler ausgestaltet, der beispielsweise aus analogen Bauteilen oder als integrierter Schaltkreis aufgebaut sein kann. Ein solcher Regler umfasst vorzugsweise ein Mittel zum Messen der Ist-Stromstärke (beispielsweise in der Zuleitung zur ersten Elektrode), ein Mittel zum Bestimmen einer Abweichung von einer vorbestimmten Soll-Stromstärke und ein Mittel zum Einstellen einer Korrektur der Spannung U entsprechend der Abweichung, insbesondere proportional zur Abweichung (Proportionalregler). Eine bevorzugte erfindungsgemäße Gleichstromabgabevorrichtung verfügt über einzeln einstellbare und regelbare Schaltkreise.

Es kann allerdings auch bevorzugt sein, eine technisch möglichst einfache erfindungsgemäße Gleichstromabgabevorrichtung zur Verfügung zu stellen. Daher ist auch eine erfindungsgemäße Gleichstromabgabevorrichtung bevorzugt, die mehrere Vorwiderstände umfasst; vorzugsweise ist jede Nadel der ersten Elektrode jeweils elektrisch leitend mit einem Vorwiderstand verbunden. Die Vorwiderstände sind Mittel zum jeweiligen Konstanthalten der Stromstärke bei der individuellen Abgabe von Gleichstrom durch Nadeln der ersten Elektrode.

Bevorzugte Vorwiderstände haben einen Widerstand von 10 kΩ bis 100 kΩ, 15 kΩ bis 80 kΩ, 20 kΩ bis 60 kΩ, 25 kΩ bis 40 kΩ und insbesondere 30 kΩ. Die Vorwiderstände sind vorzugsweise ausreichend, um Schwankungen des Hautwiderstands im Vergleich dazu unwesentlich zu machen. Es sind unveränderliche oder veränderliche Vorwiderstände einsetzbar. Vorwiderstände können mit einem oder mehreren weiteren hierin beschriebenen Mitteln zum jeweiligen Konstanthalten der Stromstärke bei der individuellen Abgabe von Gleichstrom durch (die) Nadeln der ersten Elektrode kombiniert werden, allerdings können sie auch in Abwesenheit solcher weiterer Mittel eingesetzt werden. Vorwiderstände können alternativ mit einem Mittel zum Konstanthalten der Gesamtstromstärke bei der Abgabe des Gleichstroms kombiniert werden, oder auch in Abwesenheit einen solchen Mittels eingesetzt werden.

Erfindungsgemäß bevorzugte Gleichstromquellen sind zum Beispiel Batterien. Der Begriff "Batterie" umfasst im Rahmen der vorliegenden Erfindung neben Batterien mit einer Spannung von vorzugsweise 1,2 V (etwa Nickel-Metallhydrid-Batterien) bis 1,5 V (etwa Alkali-Mangan- oder Zink-Kohle-Batterien), alleine oder in bevorzugt zwei-, drei- oder vierfacher Ausführung hintereinander geschaltet) auch Akkumulatoren und galvanische Zellen. Eine bevorzugte Batterie hat eine Spannung von 1,2 bis 1,5 V und liegt alleine oder in bevorzugt zwei-, drei- oder vierfacher Ausführung hintereinander geschaltet vor.

Weitere erfindungsgemäß bevorzugte Gleichstromquellen sind Netzteile oder Konstantstromquellen. Eine besonders bevorzugte Gleichstromquelle ist beispielsweise in dem von der neuroConn GmbH (Ilmenau, Deutschland) unter der Bezeichnung "DC-Stimulator" vertriebenen Gerät enthalten. Dieses Gerät wird im Stand der Technik zur transkraniellen Gleichstromstimulation (tDCS) des Gehirns angewendet. Es umfasst ein automatisiertes Mittel zum Konstanthalten der Stromstärke und wird in einem Kit zusammen mit zwei Schwammelektroden zum Auflegen auf den Kopf zur transkraniellen Gleichstromstimulation angeboten, nicht aber zur Elektroakupunktur. Die enthaltene Stromquelle ist unterschiedlich zu den im Stand der Technik für die Elektroakupunktur eingesetzten Stromquellen, da sie Gleichstrom statt Wechselstrom liefert und die abgegebene Stromstärke außerdem wesentlich geringer ist. Bevorzugt ist der "DC-Stimulator MC".

Vorzugsweise umfasst die erfindungsgemäße Gleichstromabgabevorrichtung einen veränderbaren inneren Widerstand. zum Einstellen der abgegebenen Stromstärke.

Erfindungsgemäß wird unter einer Nadel ein länglicher (vorzugsweise zylindrischer) Körper verstanden, dessen Länge im Verhältnis zum Durchmesser groß ist. Vorzugsweise hat eine Nadel ein zugespitztes Ende, insbesondere ein konisch zugespitztes. Die Nadel oder Nadeln zur Verwendung als erste Elektrode ist/sind vorzugsweise so ausgebildet, dass durch ihre Anwendung der menschliche oder tierische Körper nicht verletzt wird. Der Durchmesser eines einzustechenden Bereichs liegt (ohne Berücksichtigung eines zugespitzten Endes) vorzugsweise zwischen 0,1 und 0,8 mm, bevorzugt zwischen 0,2 und 0,4 mm und insbesondere bei etwa 0,3 mm, wobei ein der einzustechende Bereich vorzugsweise außerdem ein zugespitztes Ende hat; die Länge eines einzustechenden Bereichs liegt vorzugsweise zwischen 10 und 100 mm, bevorzugt zwischen 20 und 50 mm und insbesondere bei etwa 30 mm. Der Durchmesser in einem Griffbereich kann beispielsweise etwa 1-3 mm betragen, um eine einfache Verbindung mehrerer Nadeln zu ermöglichen. Bevorzugte Nadeln haben die Form bekannter Akupunkturnadeln und folgende Dimensionen: 0,2 x 15 mm, 0,25 x 40 mm, 0,3 x 30 mm, 0,3 x 100 mm, 0,35 x 50 mm und 0,35 x 100 mm.

Das Material von Nadel(n) zur Verwendung als erste Elektrode ist vorzugsweise Metall. Bevorzugte Metalle sind Edelstähle, d.h. unlegierte oder legierte Stähle mit geringem Schwefel- und Phosphorgehalt. Weitere Legierungsbestandteile sind vorzugsweise Chrom (vorzugsweise in einem Anteil von 10,5-13 Gew.-% oder höher), Nickel (vorzugsweise in einem geringen Anteil, etwa maximal 10 Gew.-%), Molybdän, Titan und/oder Niob. Bevorzugt ist 18/10 Chrom-Nickel-Stahl oder medizinischer Edelstahl. Bevorzugte Stähle sind solche, die gegen Wasser und schwache organische und anorganische Säuren beständig sind. Insbesondere bevorzugt sind nichtrostende Stähle. Weitere bevorzugte Metalle sind Silber, Gold und Platin. Optional sind die Nadeln lediglich versilbert, vergoldet bzw. platiniert. Bevorzugt sind auch Sinterwerkstoffe, beispielsweise aus Silber/Silberchlorid.

Die erste Elektrode umfasst eine Mehrzahl von Nadeln, vorzugsweise 2-20, bevorzugt 3-15, 3-12, 4-10, 5-9, 5-8, 6-9, 6-8 und insbesondere 6 oder 8. Dies erlaubt ein besonders gutes Umkreisen eines zu behandelnden Bereichs. In besonderen Ausführungsformen kann die erste Elektrode auch eine höhere Anzahl von Nadeln umfassen, was insbesondere vorteilhaft ist, falls mehr als ein zu behandelnder Bereich vorliegt.

In der erfindungsgemäßen Gleichstromabgabevorrichtung sind die Spitzen der Mehrzahl von Nadeln der ersten und optional der zweiten Elektrode vorzugsweise entlang eines im Wesentlichen kreisförmigen oder elliptischen Umfangs angeordnet. Vorzugsweise liegt die zweite Elektrode außerhalb des von den Nadeln der ersten Elektrode umschriebenen Bereichs. Diese Ausführungsformen ermöglichen besonders vorteilhafte Behandlungen.

Alternativ dazu können die Nadeln auch entlang einer Linie angeordnet sein, die beispielsweise im Wesentlichen gerade oder auch abgeknickt oder gekrümmt sein kann.

In besonderen Ausführungsformen können auch eine erste Gruppe von Nadeln und eine oder mehrere weitere Gruppe(n) von Nadeln vorgesehen sein (etwa zwei, drei, vier oder fünf Gruppen von Nadeln als erste Elektrode), was eine Behandlung mehr als eines zu behandelnden Bereichs erlaubt. In diesen Ausführungsformen ist es bevorzugt, wenn die Spitzen der Nadeln der ersten Gruppe entlang eines im Wesentlichen kreisförmigen oder elliptischen Umfangs angeordnet sind und die Spitzen der Nadeln der weiteren Gruppe(n) (jeweils) ebenfalls entlang eines im Wesentlichen kreisförmigen oder elliptischen Umfangs angeordnet sind. Die zweite Elektrode liegt hierbei vorzugsweise außerhalb der von den Nadeln umschriebenen Bereiche. Bevorzugt ist es, wenn eine gegebenenfalls vorhandene elektrisch leitende Verbindung der Nadeln entlang des jeweiligen (kreisförmigen oder elliptischen) Umfangs der einzelnen Gruppen von Nadeln ausgebildet ist (Reihenschaltung der Nadeln der jeweiligen Gruppe) und/oder die Gruppen durch jeweils eine einzelne elektrisch leitende Verbindung in Reihe geschaltet sind.

Alternativ dazu können die Spitzen der Nadeln der ersten Gruppe entlang einer Linie angeordnet sein, die beispielsweise im Wesentlichen gerade oder auch abgeknickt oder gekrümmt ist, und die Spitzen der Nadeln der weiteren Gruppe(n) können (jeweils) ebenfalls entlang einer Linie angeordnet sein, die beispielsweise im Wesentlichen gerade oder auch abgeknickt oder gekrümmt ist, wobei vorzugsweise die Nadeln der einzelnen Gruppen jeweils in Reihe geschaltet sind und/oder die Gruppen durch jeweils eine einzelne elektrisch leitende Verbindung in Reihe geschaltet sind.

Vorzugsweise ist die erste Elektrode (Elektrode zur Verwendung im zu behandelnden Bereich) als Kathode (negativer Pol) und die zweite Elektrode als Anode (positiver Pol) ausgebildet. Hierdurch werden die Behandlungsmöglichkeiten unter Einsatz der erfindungsgemäßen Gleichstromabgabevorrichtung optimiert.

Die zweite Elektrode ist vorzugsweise als flächige Elektrode ausgebildet (Variante A). Eine solche Elektrode ist als Oberflächenelektrode einsetzbar und ist vorzugsweise angepasst an eine Anbringung auf der Körperoberfläche, beispielsweise durch Ausgestaltung als Klebeelektrode (wie oben definiert). Bevorzugt ist eine Elektrode, die in einem Klebestreifen eingearbeitet ist oder anderweitig mit einem Klebestreifen verbunden ist. Eine Klebewirkung kann auch durch Elektrodengel oder Elektrodenpaste vermittelt werden. Vorzugsweise ist das Material für die zweite Elektrode ausgewählt aus der Gruppe bestehend aus leitfähiger Gummi, leitfähiges Textil, leitfähiger Kunststoff, Schwamm (zu tränken mit beispielsweise Wasser oder NaCI-Lösung), Sinterwerkstoff (beispielsweise Silber/Silberchlorid) und Metall (beispielsweise Edelstahl, Silber, Gold und/oder Platin).

Bevorzugte Größen einer flächigen zweiten Elektrode sind 25 cm² bis 200 cm², insbesondere 50 cm² bis 100 cm²,

Ohne an eine bestimmte Theorie gebunden zu sein, wird angenommen, dass eine Verwendung von Silber/Silberchlorid-Elektroden vor allem in chloridhaltigem Medium wie dem Körpermilieu das Kontaktpotenzial (junction potential) am Metall/Elektrolyt-Übergang stabilisiert, so dass die Stromabgabe noch besser kontrollierbar wird.

Die zweite Elektrode umfasst optional eine Mehrzahl von flächigen Gebilden, beispielsweise zwei, drei, vier oder fünf.

Daneben ist es auch möglich, die zweite Elektrode als Nadel auszubilden (Variante B), wobei für bevorzugte Ausgestaltungen dieser Nadel das zu den Nadeln der ersten Elektrode Gesagte gilt.

Besonders bevorzugte Kombinationen aus erster und zweiter Elektrode sind wie folgt: nadelförmige erste Elektrode im Kombination mit nadelförmiger zweiter Elektrode und nadelförmige erste Elektrode im Kombination mit flächiger zweiter Elektrode. Es gilt das oben zu nadelförmigen Elektroden bzw. flächigen Elektroden Gesagte. Insbesondere umfasst beispielsweise eine nadelförmige zweite Elektrode optional eine Mehrzahl von Nadeln (beispielsweise zwei, drei, vier oder fünf oder auch so wie für die erste Elektrode beschrieben) und/oder umfasst eine flächige zweite Elektrode optional eine Mehrzahl von flächigen Gebilden (beispielsweise zwei, drei, vier oder fünf).

Gemäß einer alternativen Ausführungsform sind beide Elektroden in einem einzelnen Gebilde vereint (mehrpolige Nadel). Dies ist beispielsweise vorteilhaft bei der Behandlung eines räumlich eng umgrenzten Gebiets oder bei Patienten, die die Applikation von Elektroden generell schlecht tolerieren. So kann erfindungsgemäß eine Nadel die erste Elektrode und die zweite Elektrode entlang ihrer Längserstreckung hintereinander aufweisen, beispielsweise die Kathode näher am einzustechenden Ende als die Anode oder auch die Anode näher am einzustechenden Ende als die Kathode. Ferner ist die zweite Elektrode hierbei entweder kontinuierlich oder diskontinuierlich. Beispielsweise kann die Oberfläche der Kathode und/oder der Anode optional als ein oder mehrere Zylindermäntel ausgebildet sein. Vorzugsweise ist in diesem Fall die Oberfläche der ersten Elektrode als Mehrzahl von Zylindermänteln ausgebildet und die Oberfläche der zweiten Elektrode als Zylindermantel oder Mehrzahl von Zylindermänteln.

Vorzugsweise sind die Elektroden, die Mittel zum Verbinden der Elektroden mit der Gleichstromquelle und/oder die Anschlüsse der Gleichstromquelle entsprechend der Polarität gekennzeichnet, beispielsweise durch Farbe oder Form, Symbole wie + und -, Ziffern oder Buchstaben.

Bevorzugt ist eine physiologische akzeptable Stromstärke bzw. eine die Zellen des Körpergewebes nicht schädigende Stromstärke. Die maximale Gesamtstromstärke des Gleichstroms beträgt bei der bevorzugten Variante A mit flächiger zweiter Elektrode vorzugsweise 2000 µA, bevorzugt 1000, 700, 500, 400, 300, 250, 200, 150 oder 100 µA. Bei der Variante B mit einer Nadel als zweite Elektrode beträgt die maximale Gesamtstromstärke des Gleichstroms vorzugsweise 1000, 750, 500, 250, 200, 150, 100, 50, 25 oder 5 µA. Unter "Gesamtstromstärke" wird die Summe der Stromstärken verstanden, die von den einzelnen Nadeln der ersten Elektrode abgegeben werden.

Die bevorzugte minimale Gesamtstromstärke des Gleichstroms liegt bei 10, 20, 30, 40 oder 50 µA (Variante A) oder 1, 1,5, 2 oder 2,5 µA (Variante B). Besonders bevorzugt sind Bereiche für die Stromstärke von 10-800 µA, 10-600 µA, 10-400 µA, 10-250 µA, 20-250 µA, 10-200 µA, 20-200 µA, 10-150 µA, 20-150 µA, 30-150 µA, 20-100 µA, 30-100 µA, 40-100 µA und 50-100 µA (Variante A) bzw. Bereiche von 1-100 µA, 1-50 µA, 1-25 µA, 1,5-20 µA, 2-15 µA, 2-10 µA und 2,5-5 µA (Variante B). Die erfindungsgemäße Gleichstromabgabevorrichtung umfasst vorzugsweise ein Mittel zum Einstellen der Gesamtstromstärke und insbesondere ein Mittel zum Einstellen einer minimalen und/oder maximalen Gesamtstromstärke, in allen Fällen vorzugsweise fernsteuerbar.

Die Einzelstromstärken können identisch oder verschieden sein. Bevorzugt sind die Einzelstromstärken identisch, insbesondere im Zeitmittel, oder aber der Faktor, der sich durch Division der größten Einzelstromstärke durch die kleinste Einzelstromstärke ergibt, insbesondere jeweils im Zeitmittel, ist nicht größer als 2, 1,9, 1,8, 1,7, 1,6, 1,5, 1,4, 1,3, 1,25, 1,2, 1,18, 1,15, 1,12, 1,1, 1,08 oder 1,05. In anderen Fällen können auch verschiedene Einzelstromstärken bevorzugt sein; für solche Fälle ist es vorteilhaft, wenn die Einzelstromstärken individuell regulierbar sind.

Vorzugsweise beträgt die Schwankung einer Einzelstromstärke um ihren konstanten Wert maximal 50% des konstanten Werts, vorteilhafterweise maximal 40%, 30%, 25%, 20%, 15%, 12%, 10%, 9%, 8%, 7%, 6%, 5%, 4%, 3%, 2% oder 1%. Vorzugsweise ist dies bei allen Einzelstromstärken der Fall.

Bevorzugte maximale und minimale Einzelstromstärken ergeben sich aus einer bevorzugten maximalen bzw. minimalen Gesamtstromstärke und einer bevorzugten Anzahl von Nadeln der ersten Elektrode. Bevorzugte maximale Einzelstromstärken liegen beispielsweise bei 6 µA, 8 µA, 10 µA, 12 µA, 14 µA, 16 µA, 18 µA, 20 µA, 30 µA, 40 µA, 50 µA, 60 µA, 70 µA, 80 µA, 90 µA, 100 µA, 110 µA, 120 µA, 130 µA, 140 µA, 150 µA, 160 µA, 170 µA, 180 µA, 190 µA, 200 µA, 250 µA, 300 µA, 350 µA, 400 µA, 450 µA, 500 µA, 550 µA, 600 µA, 650 µA oder 700 µA (Variante A) und bei 0,3 µA, 0,4 µA, 0,5 µA, 0,6 µA, 0,7 µA, 0,8 µA, 0,9 µA, 1 µA, 2 µA, 3 µA, 4 µA, 5 µA, 6 µA, 7 µA, 8 µA, 9 µA, 10 µA, 20 µA, 30 µA, 40 µA, 50 µA, 60 µA, 70 µA, 80 µA, 90 µA, 100 µA, 110 µA, 120 µA, 130 µA, 140 µA, 150 µA, 160 µA, 170 µA, 180 µA, 190 µA, 200 µA, 250 µA, 300 µA oder 350 µA (Variante B).

Die Stromdichte, definiert als abgegebene Stromstärke, bezogen auf die von einer Nadel kontaktierte Fläche, beträgt vorzugsweise maximal 10 µA/mm², bevorzugt maximal 7 µA/mm², maximal 5 µA/mm², maximal 3 µA/mm², maximal 2,5 µA/mm², maximal 2 µA/mm², maximal 1,5 µA/mm², maximal 1 µA/mm² oder maximal 0,5 µA/mm² . Die elektrische Spannung beim Einsatz der Gleichstromabgabevorrichtung zur Behandlung des menschlichen oder tierischen Körpers beträgt vorzugsweise maximal 24 V, 20 V, 18 V, 16 V, 14 V, 13 V, 12 V, 11 V, 10 V, 9 V, 8 V, 6 V 4,8 V, 4,5 V, 3,6 V, 3 V, 2,4 V, 1,5 V oder 1,2 V. Hierdurch wird sichergestellt, dass schädliche Einwirkungen auf den Körper vermieden werden. Die erfindungsgemäße Gleichstromabgabevorrichtung umfasst vorzugsweise ein (insbesondere fernsteuerbares) Mittel zum Einstellen einer maximalen Spannung. Ferner umfasst sie vorzugsweise ein (insbesondere fernsteuerbares) Mittel zum Einstellen einer maximalen Ladung.

Die Stärke des elektrischen Feldes liegt vorzugsweise im Bereich von 10-2500 mV/mm, insbesondere von 200-1500 mV/mm. Die Felddichte kann in der Umgebung nadelförmiger Elektroden noch höher sein, was ein bevorzugtes Behandlungsprinzip unter Verwendung der erfindungsgemäßen Gleichstromabgabevorrichtung ist. Stärke und Verlauf des elektrischen Feldes in der Umgebung nadelförmiger Elektroden richten die Wirkung bei Einsatz der erfindungsgemäßen Gleichstromabgabevorrichtung hauptsächlich in das Gebiet, in dem die Elektrode appliziert ist bzw. in ihre unmittelbare Nachbarschaft. In der unmittelbaren Umgebung einer nadelförmigen Elektrode nimmt die Feldstärke in orthogonaler Richtung exponentiell ab.

Optional umfasst die erfindungsgemäße Gleichstromabgabevorrichtung ferner ein Mittel zur zeitlichen Steuerung, mit dem mehrere Zeiträume zur Abgabe des Gleichstroms vorbestimmt werden können. Im einfachsten Fall ist dies eine elektronische schaltbare Unterbrechung der elektrisch leitenden Verbindung zwischen Kathode, Gleichstromquelle und Anode. Vorzugsweise ist das Mittel zur zeitlichen Steuerung mit einem Mittel zur Einstellung eines minimalen Zeitraums verknüpft, wobei Letzteres vorzugsweise eine Bestimmung eines minimalen Zeitraums von 1 s, 10 s, 1 min, 2 min, 5 min, 10 min, 20 min oder 30 min erlaubt. Vorzugsweise ist das Mittel zur zeitlichen Steuerung fernsteuerbar. Gemäß einer bevorzugten Ausführungsform ist das Mittel zur zeitlichen Steuerung ferner mit einem (vorzugsweise fernsteuerbaren) Mittel zur Bestimmung eines maximalen Zeitraums verknüpft, wobei Letzteres vorzugsweise eine Bestimmung eines maximalen Zeitraums von 2 h, 1 h, 50 min, 40 min, 30 min, 20 min, 10 min, 5 min oder 2 min erlaubt.

Vorzugsweise umfasst die erfindungsgemäße Gleichstromabgabevorrichtung ein (insbesondere fernsteuerbares) Mittel zum Auflaufen und Auslaufen (Ramping) der Stromstärke. Ein solches Mittel erlaubt zu Beginn der Behandlung ein Ansteigen der Stromstärke von Null bis zum Sollwert innerhalb eines vorbestimmbaren Zeitraums (mit einer bevorzugten Länge von 1-60 Sekunden, besonders bevorzugt 5-45 Sekunden und insbesondere 10-30 Sekunden) und am Ende der Behandlung ein Abfallen vom Sollwert bis auf null innerhalb eines vorbestimmbaren Zeitraums (mit einer bevorzugten Länge von 1-60 Sekunden, besonders bevorzugt 5-45 Sekunden, weiter bevorzugt 10-30 Sekunden und insbesondere 15 Sekunden). Ein langsames Auflaufen und Auslaufen der Stromstärke ist vorteilhaft, weil sonst - beim sprunghaften An- oder Ausschalten des Stromes - das behandelte Individuum ein unangenehmes Zucken oder ein Stromschlaggefühl verspürt.

Optional umfasst die erfindungsgemäße Gleichstromabgabevorrichtung ein (vorzugsweise fernsteuerbares) Mittel zum Umschalten der Polarität der Elektroden während einer Behandlung. Dieses ist vorzugsweise mit einem Mittel zur zeitlichen Steuerung verknüpft, so dass es beispielsweise möglich ist, die Polarität jede Sekunde, alle 10 s, jede Minute, alle 2 min, alle 5 min oder alle 10 min umzuschalten.

Die Gleichstromabgabevorrichtung ist optional auf einen Testmodus einstellbar, in dem eine konstante Spannung von etwa 1-8 V, 2-6 V oder 3-5 V abgegeben wird. Damit kann überprüft werden, ob die Elektroden (beispielsweise insbesondere alle Nadeln) elektrisch korrekt verkoppelt sind oder nicht, oder ob in irgendeinem Kabel ein okkulter Kabelbruch vorliegt. Vorzugsweise umfasst die Gleichstromabgabevorrichtung einen Signalgeber (etwa einen Tonerzeuger), der anzeigt, wenn ein korrekter Stromfluss entsteht. Aus einer Abwesenheit des Signals kann geschlossen werden, dass die Kopplerkette unterbrochen ist.

Ein Signalgeber ist vorzugsweise auch dafür einsetzbar, den Anfang und/oder das Ende der Behandlung anzuzeigen. Ferner ist ein Signalgeber vorzugsweise dafür einsetzbar anzuzeigen, falls während einer Behandlung der Stromfluss unterbrochen wird oder die Impedanz des Patienten zu hoch ist, insbesondere in Kombination mit einem Abschaltmittel.

Mit Hilfe des Testmodus können auch die einzelnen Elektroden (z.B. Nadeln) direkt stimuliert werden und aus der Reaktion des Patienten (Muskelzuckung bzw. in nicht kontraktilem Gewebe Schmerz) auf die richtige Positionierung der Elektrode (insbesondere Nadel) geschlossen werden, wie unten näher beschrieben.

Das erfindungsgemäße Kit zum Herstellen einer Gleichstromabgabevorrichtung umfasst vorzugsweise zusätzlich eine Anleitung zur therapeutischen oder kosmetischen Behandlung des menschlichen oder tierischen Körpers, wobei die Behandlung vorzugsweise ist wie im Folgenden genauer erläutert. Eine bevorzugte Ausführungsform der erfindungsgemäßen Gleichstromabgabevorrichtung umfasst eine Gleichstromquelle, eine erste Elektrode sowie eine zweite Elektrode zum Verbinden mit der Gleichstromquelle und ein oder mehrere Mittel zum jeweiligen Konstanthalten der Stromstärke bei der individuellen Abgabe des Gleichstroms durch die Nadeln der ersten Elektrode, wobei die erste Elektrode als Mehrzahl nicht unmittelbar miteinander elektrisch leitend verbundener Nadeln ausgebildet ist und die zweite Elektrode als flächige Elektrode ausgebildet ist. Bevorzugt ist dabei eine maximale Gesamtstromstärke des Gleichstroms von 2000 µA, weiter bevorzugt 1000 µA, besonders bevorzugt 700 µA und insbesondere 500 µA. Selbstverständlich sind auch die oben aufgeführten niedrigeren Stromstärken mit dieser Ausführungsform kombinierbar. Außerdem bevorzugt ist dabei eine erste Elektrode, die 2-20 Nadeln umfasst. Die Einzelstromstärken sind hierbei vorzugsweise identisch.

Die erfindungsgemäße Gleichstromabgabevorrichtung wird vorzugsweise bei der Behandlung von Entzündungen und/oder Schmerzen, insbesondere der Muskeln, Nerven, Sehnen oder Knochen eingesetzt.

Bei der Behandlung ist eine Applikation von Dauerstrom gegenüber einer Applikation von gepulstem Strom bevorzugt. Alternativ möglich sind eine undulierende Stromstärke wie oben beschrieben oder eine sich nicht periodisch verändernde Stromstärke (die zu jedem Zeitpunkt im Wesentlichen den gleichen Wert hat). Vorzugsweise wird die jeweilige Stromstärke bei der individuellen Abgabe des Gleichstroms durch die Nadeln der ersten Elektrode (oder der Wert, um den die jeweilige Stromstärke unduliert) konstant gehalten, insbesondere auch in dem Fall, dass sich ein Widerstand verändert, der an einer oder mehreren der Nadeln der ersten Elektrode (jeweils) anliegt.

Die Dauer einer bevorzugten Behandlung liegt zwischen 1 min und 2 h, 5 min und 1 h, 10 min und 50 min, 20 min und 40 min und vorzugsweise bei 30 min. Bevorzugt umfasst die Behandlung eine Gesamtstromabgabezeit von 60 min, 45 min, 30 min oder 20 min. Vorzugsweise erfolgt die Stromabgabe bei der Behandlung ohne Unterbrechung. Gemäß einer alternativen Ausführungsform kann die Behandlung jedoch auch mehrere vorbestimmte Zeiträume (beispielsweise 2, 3, 4, 5, 6 oder mehr vorzugsweise gleich lange Zeiträume) umfassen, während derer der Gleichstrom appliziert wird, wobei zwischen den Zeiträumen vorzugsweise eine Pause von 1 s bis 5 min, 10 s bis 3 min oder 30 s bis 1 min vorgesehen ist. Alternativ kann ein An- und Abschalten des Gleichstroms mit einer Frequenz von 0,01-1 Hz, vorzugsweise 0,02 bis 0,2 Hz und insbesondere 0,05 bis 0,1 Hz erfolgen. Diese Zeiträume bzw. das An- und Abschalten werden gesteuert durch das gegebenenfalls vorgesehene Mittel zur zeitlichen Steuerung (siehe oben). Vorzugsweise erfolgt zu Beginn und am Ende der Behandlung ein langsames Auf- bzw. Auslaufen (Ramping) der Stromstärke, etwa über einen Zeitraum von jeweils 1-60 Sekunden, bevorzugt 5-45 Sekunden und insbesondere 10-30 Sekunden und besonders bevorzugt 15 Sekunden.

Die Behandlung erfolgt vorzugsweise innerhalb eines Tages, insbesondere innerhalb eines Zeitraums von 4, 3 oder 2 Stunden. Je nach Behandlungsansatz ist es bevorzugt, die Behandlung einmalig oder wiederholt (insbesondere ein-, zwei- oder dreimal pro Woche oder täglich) durchzuführen.

Im Vorfeld der Stromapplikation wird der zu behandelnde schmerzende/entzündete Bereich vorzugsweise eingekreist und so lokalisiert, bevorzugt durch tiefes Tasten bis zum Knochen von allen Seiten her. In der Terminologie der TCM wird ein entsprechender druckschmerzhafter Punkt "Ahshi" genannt. Der zu behandelnde Bereich wird nach optionalem Desinfizieren mit einer Elektrode versehen, bevorzugt einer oder mehreren Nadeln (vorzugsweise durch Einstecken) und insbesondere mit einer Mehrzahl von Nadeln umschrieben (insbesondere kreisförmig oder elliptisch). Umschreiben bedeutet hierbei vorzugsweise, dass die Nadeln entlang des Randes dieses Bereichs gesteckt werden. Weitere Möglichkeiten bestehen darin, die Nadeln einige Millimeter außerhalb des Randes oder aber im Inneren des Bereichs zu stecken. Das genannte Einstecken der Nadel(n) erfolgt vorzugsweise möglichst tief (zum Beispiel in die Subkutis, in einen Muskel, in die Ligamente oder Sehnen, unter das Acromion, auf die Facetten der Wirbelsäule oder auf die Knochenhaut), optional nach Lokalanästhesie. Alternativ dazu kann der zu behandelnde Bereich auch durch Anordnen einer Mehrzahl von Nadeln entlang einer im Wesentlichen geraden Linie mit Nadeln versehen werden, wobei die Linie diesen Bereich schneidet oder tangieret oder auch außerhalb des Bereichs liegt.

Die zweite Elektrode wird am oder im Körper positioniert, und zwar vorzugsweise außerhalb eines von den Nadeln der ersten Elektrode umschriebenen Bereichs. Insbesondere wird die zweite Elektrode in einem anderen Körperbereich als die erste Elektrode positioniert. Vorzugsweise wird die zweite Elektrode (Variante A: flächige Elektrode) oberhalb von großen Muskelgruppen oder Fettschichten positioniert, damit nicht einzelne Nerven durch sie stimuliert werden. Eine zweite Elektrode gemäß Variante B (nadelförmige Elektrode) wird vorzugsweise intramuskulär appliziert.

Vorzugsweise wird vor der eigentlichen Behandlung durch kurzzeitiges Anlegen eines Stroms sichergestellt, dass (eine) eingesteckte Nadel(n) nicht in der Nähe von Nervenwurzeln liegt/liegen, damit es bei der Behandlung nicht zu einer Schmerzreaktion oder einer motorischen Reaktion kommt. Andernfalls müsste(n) die Nadel(n) etwas zurückgezogen oder an einer anderen Stelle positioniert werden.

Alternativ dazu kann in bestimmten Ausführungsformen ein Muskelzucken auch gezielt genutzt werden, um die Positionierung einer Nadel zu überprüfen. Durch elektrischen Strom kann ein Muskelzucken ausgelöst werden, beispielsweise indem Elektroden während einer Stromabgabe an eingestochene Nadeln gehalten werden. Je stärker dieses Zucken, desto besser ist die Nadel positioniert. Wie oben bereits erwähnt, ist es möglich, etwa unter Verwendung des beschriebenen Testmodus die einzelnen Elektroden (z.B. Nadeln) direkt zu stimulieren und aus der Reaktion des Patienten auf die richtige Positionierung der Elektrode (insbesondere Nadel) zu schließen. So bewirkt eine in einen entzündeten schmerzhaften Muskel eingesetzte Nadel bei direkter Teststimulation eine im Vergleich zu einer in einen nicht entzündeten Muskel eingestochenen Nadel eine verstärkte Muskelzuckung. Bei in nicht kontraktiles Gewebe eingestochenen Nadeln verspürt der Patient bei direkter Teststimulation einen vermehrten Schmerz (Brennen) an der Nadel im entzündeten Gewebe im Vergleich zu einem nicht entzündeten Gewebe.

Optional können auch mehrere separat regelbare Stromquellen eingesetzt werden.

Es ist möglich, dass die durch die erfindungsgemäße Gleichstromabgabevorrichtung erzeugten elektrischen Felder zusätzlich zu ihrer antiphlogistischen und analgetischen Wirkung eine rekonstruierende Wirkung aufweisen, beispielsweise über eine Förderung des Gefäßwachstums, unter anderem über die Ausschüttung von VEGF und einen Einfluss auf Endothelzellen. Weiterhin ist es möglich, dass sie zu einer Bewegung und Neuanordnung von Zellmembranrezeptoren führen, die Teilungsrate von bestimmten Zellen erhöhen, die Zellwanderung von Epithelzellen (insbesondere zur Kathode hin) und die Wundheilung beschleunigen. Auch ist es denkbar, dass dadurch die periphere Nervenregeneration nach Rückenmarkstrauma durch Wachstum zur Kathode hin beschleunigt werden kann, die dann vorzugsweise kopfwärts liegen würde.

Ohne an eine bestimmte Theorie gebunden zu sein, ist die Wirkung der Anwendung der erfindungsgemäßen Gleichstromabgabevorrichtung auf den menschlichen oder tierischen Körper zurückzuführen auf die unmittelbare Einwirkung des verabreichten elektrischen Stroms bzw. des angelegten elektrischen Feldes auf das betroffene Gewebe bzw. die betroffenen Zellen. Eine Erklärung ist beispielsweise eine Veränderung der elektrischen Erregbarkeit von Zellen, insbesondere Nervenzellen (De- oder Hyperpolarisation), womöglich über eine Wirkung auf Kationenkanäle oder über eine temporäre Verschiebung des lonengleichgewichts zwischen Intrazellulär- und Extrazellulärraum. Hierfür käme insbesondere ein Efflux von Kaliumionen und anderen Kationen in den Extrazellulärraum infrage, wodurch eine lokale antiphlogistische und analgetische Wirkung erklärt werden könnte. Auch eine Regeneration von aseptischen Wunden oder degenerativ veränderten Gewebeanteilen oder eine Wanderung von Zellen im elektrischen Feld könnte (mit)ursächlich für die beobachtete Wirkung sein. Es wird angenommen, ohne an eine Theorie gebunden zu sein, dass der verabreichte elektrische Strom bzw. das angelegte elektrische Feld fundamentale Entzündungsprozesse, Prozesse der Schmerzentstehung und/oder der Geweberegeneration in den Zellen und im Gewebe direkt und lokal beeinflusst. Dadurch werden grundlegende elektrophysiologische/neurophysiologische Mechanismen beeinflusst.

Die Stromstärke und Spannung sind dabei um ein Vielfaches (mehrere Zehnerpotenzen) kleiner als bei bekannten Elektroakupunkturgeräten. Insbesondere wird Gleichstrom und nicht Wechselstrom eingesetzt.

Bei bekannten medizinischen Elektrostimulationsgeräten beruht das Wirkprinzip auf einer hohen Intensität der Spannung und/oder des Stroms. Zum Beispiel soll bei galvanischen Bädern oder bei der Kauterisation eine Erhitzung des Gewebes bewirkt werden, oder bei der TENS (Gate Gontrol Theorie) eine überschwellige Rezeptorreizung zur Schmerzunterdrückung. Im Gegensatz hierzu arbeitet die erfindungsgemäße Gleichstromabgabevorrichtung bei kleinsten Spannungen, Stromstärken und elektrischen Feldern, wodurch insbesondere eine entzündungshemmende, schmerzhemmende und/oder regenerative Wirkung erzeugt wird.

Nach Einstechen einer Nadel wird zwischen dem negativen Pol an der Nadel und einer auf der Haut aufgeklebten breitflächigen Elektrode beispielsweise eine Potenzialdifferenz von 100-300 mV gemessen. Durch schnelles manuelles Drehen der Nadel kann sich die Potenzialdifferenz erhöhen, was in erster Linie auf die Beeinflussung des Elektroden-Kontaktpotenzials zurückzuführen ist und nachfolgend logarithmisch wieder auf den Ausgangswert absinken. Das Einstechen von Nadeln und ihre manuelle Stimulation sind grundlegende Techniken der analgetischen Akupunktur.

Gegenstand der vorliegenden Offenbarung ist ferner die erfindungsgemäße Gleichstromabgabevorrichtung zur Anwendung bei der Behandlung folgender Beeinträchtigungen oder Beschwerden: Migräne, Spannungskopfschmerz (z.B. migräneartiger Spannungskopfschmerz), Neuralgien (z.B. post-Zoster-Neuralgie, Occipitalisneuralgie, Trigeminusneuralgie, Neuralgie des Nervus femoralis, besonders postoperativ), Herpes Zoster-Schmerz, neuropathischer post-Zoster Schmerz, Bing-Horton-Syndrom, Tinnitus, Allergien, entzündliche Zeichen bei Allergie, HWS-Syndrom, BWS-Syndrom, LWS-Syndrom, chronischer Kreuzschmerz, Spinalkanalstenose, Zervikobrachialgie, Ischialgie, Radikulitis, Periarthritis humero scapularis, Schmerzen bei Arthrose, Gonarthrose, Arthritis (soweit nicht systemisch bedingt), Tendinitis (wie Tennisarm, Golfersarm (Epikondylitis lateralis-, medialis), Tendovaginitis, Ansatztendinosen, Achillodynie, Fersensporn, Hautrötung, Hautentzündung, Seborrhöe, Psoriasis, seborrhoische, erythematöse und/oder psoriatische Erscheinungen, Akne, Haarausfall (z.B. Alopezie), durch lokale Reizzustände verursachte Bewegungseinschränkungen vor allem der Haut wie lokale Verhärtungen und Spannungen. Entsprechendes gilt für die Verwendung der erfindungsgemäßen Gleichstromabgabevorrichtung zur Behandlung der genannten Indikationen, für die Verwendung der erfindungsgemäßen Gleichstromabgabevorrichtung zur Herstellung einer therapeutischen Vorrichtung zur Behandlung der genannten Indikationen und für das erfindungsgemäße Verfahren zur Behandlung von Entzündungen und/oder Schmerzen.

Die erfindungsgemäße Gleichstromabgabevorrichtung erzeugt vorzugsweise eine langfristige, sich von Behandlung zu Behandlung aufbauende regenerative Wirkung, beispielsweise bei chronischen Tendinosen oder chronischen neuropathischen Schmerzen.

Vorzugsweise sind folgende Behandlungen ausgeschlossen: Behandlung eines Haarfollikels, Behandlung offener Wunden und Behandlung einer Hautschädigung. Gemäß einem weiteren Aspekt betrifft die vorliegende Erfindung die Verwendung der erfindungsgemäßen Gleichstromabgabevorrichtung zur kosmetischen Behandlung des menschlichen oder tierischen Körpers.

Außerdem betrifft die vorliegende Erfindung gemäß einem weiteren Aspekt ein Verfahren zum Herstellen einer Gleichstromabgabevorrichtung umfassend die folgenden Schritte: Bereitstellen eines Kits (gemäß dem dritten Aspekt der vorliegenden Offenbarung), Bereitstellen einer Gleichstromquelle, Bereitstellen der Mehrzahl von Nadeln zum Bilden der ersten Elektrode (wobei die Spitzen einer Mehrzahl von Nadeln vorzugsweise entlang eines im Wesentlichen kreisförmigen oder elliptischen Umfangs oder entlang einer Linie angeordnet werden, im Wesentlichen gerade oder auch abgeknickt oder gekrümmt ist), Bereitstellen der flächigen Elektrode oder der Nadel oder unmittelbares elektrisch leitendes Verbinden der Mehrzahl von Nadeln zum Bilden der zweiten Elektrode (wobei die Spitzen einer Mehrzahl von Nadeln vorzugsweise entlang eines im Wesentlichen kreisförmigen oder elliptischen Umfangs oder entlang einer Linie angeordnet werden, im Wesentlichen gerade oder auch abgeknickt oder gekrümmt ist und die unmittelbare elektrisch leitende Verbindung vorzugsweise entlang des Umfangs erfolgt), Verbinden der ersten Elektrode mit der Gleichstromquelle und Verbinden der zweiten Elektrode mit der Gleichstromquelle.

Ein weiterer Aspekt der vorliegenden Offenbarung ist die erfindungsgemäße Gleichstromabgabevorrichtung zur Anwendung in einem Verfahren zur therapeutischen Behandlung des menschlichen oder tierischen Körpers bzw. die Verwendung der erfindungsgemäßen Gleichstromabgabevorrichtung in einem Verfahren zur therapeutischen Behandlung des menschlichen oder tierischen Körpers.

Schließlich betrifft die vorliegende Offenbarung ein durch die erfindungsgemäße Gleichstromabgabevorrichtung erzeugbares elektrisches Feld. Das Feldmaximum liegt hierbei um den Nadelkorpus und eine Nadelspitze.

Weiterhin betrifft die vorliegende Offenbarung ein solches elektrisches Feld zur Anwendung in einem Verfahren zur therapeutischen Behandlung des menschlichen oder tierischen Körpers, insbesondere ein solches elektrisches Feld zur Behandlung von Entzündungen und/oder Schmerzen, insbesondere der Muskeln, Nerven, Sehnen oder Knochen oder zur Behandlung einer der oben genannten Indikationen.

### Beispiele

Alle in den nachfolgenden Beispielen enthaltenen Schmerzangaben wurden mit einer VAS (visuellen Analogskala) bestimmt und sind in Prozent des Ausgangswertes angegeben.

Behandlungen, bei denen keine Konstanthaltung der Stromstärke der einzelnen Nadeln stattfand sind ausdrücklich so gekennzeichnet; diese sind zu Vergleichszwecken geschildert. Alle anderen Behandlungen fanden unter Konstanthaltung der Stromstärke der einzelnen Nadeln statt; unabhängig davon, ob ausdrücklich darauf hingewiesen ist.

### Beispiel 1: Schmerzen im Kniegelenk

Seit 5 Monaten Schmerzen im medialen Kapselbereich des rechten Kniegelenkes. Schmerzareal ca. 2 cm lang und 0,5 cm breit. Platzieren von 3 Nadeln, 0,3 x 30 mm, Stromstärke je Nadel 45 µA, ± 10%, Gesamtstromstärke 135 µA. 30 min Stimulationsdauer. Direkt nach Behandlungsende ca. 40% Besserung, nach ca. 2 h schmerzfrei. Dauer der Wirkung 4 Tage. Dann leichtes Rezidiv. Erneute Behandlung in gleicher Weise. Nach einem Tag vollständige und anhaltende Beschwerdefreiheit.

### Beispiel 2: Post-Zoster-Neuralgie

Patient, männlich, 82 Jahre. Nach einer Herpes Zoster-Erkrankung jetzt seit 4 Jahren stärkster neuropathischer Schmerz und Kribbelparästhesien links thorakal Ausdehnung von der BWS bis hin zur Mammille. Umstechen des Schmerzareals mit 0,3 x 30 mm Nadeln subkutan, insgesamt 16 Nadeln. Stimulation mit 20 µA je Nadel - Gesamtstromstärke 320 µA, keine Besserung. Erneute Therapie mit 60 µA pro Nadel - Gesamtstromstärke 960 µA. Direkt nach der Therapie Schmerzerleichterung. Danach über eine Woche lange 50% Schmerzlinderung, dritte Behandlung erneut mit 60 µA je Nadel, danach innerhalb von 2 h weitere Besserung auf insgesamt 80% Schmerzlinderung. Nach weiterer Behandlung 90% Besserung.

### Beispiel 3: Fersenschmerz mit Achillessehnenansatztendinose

Patientin, 34 Jahre, seit 6 Monaten Fersenschmerz mit Knochenmarksödem und zusätzlich Schmerzen mit leichten entzündlichen Veränderungen im Ansatzbereich der Achillessehne am Kalkaneus. Platzierung von 3 Nadeln 0,35 x 50 mm im Bereich der Ferse sowie 5 Nadeln 0,15 x 20 mm im Bereich der Achillessehne. Stimulation mit insgesamt 240 µA ohne Konstanthaltung der Stromstärke der einzelnen Nadeln. Am nächsten Tag Besserung des Fersenschmerzes, aber keine Veränderung an der Achillessehne. Erneute Behandlung, diesmal mit Konstanthaltung jeder einzelnen Nadel auf 30 µA, Gesamtstimulation mit 240 µA. Nach 3 h Besserung des Fersenschmerzes und auch des Achillessehnenschmerzes um 80%.

### Beispiel 4: Bizepssehnenanriss, Zustand nach Operation

Handballer, 24 Jahre. Über 6 Monate nach Schulteroperation weiterhin Schmerzen in der Bizepssehne des rechten Oberarms. Jobes Test positiv. Spiel- und trainingsunfähig. Erste Behandlung mit insgesamt 120 µA auf 5 Nadeln 0,25 x 40 mm, ohne Konstanthaltung der Stromstärke der einzelnen Nadeln. Keine Besserung. Zweite Behandlung mit 240 µA auf 8 Nadeln 0,35 x 50 mm, ohne Konstanthaltung der Stromstärke der einzelnen Nadeln, keine Besserung. Dritte Behandlung mit 50 µA je Nadel, mit Konstanthaltung der Stromstärke jeder einzelnen Nadel, insgesamt 8 Nadeln 0,35 x 50 mm, Gesamtstrom 450 µA. Nach 3 h subjektiv besser, am nächsten Tag 50% Besserung. Wiederholung der letzten Behandlung insgesamt 3 Mal, danach wieder sportartspezifisches Training möglich. Besserung insgesamt 90%.

### Beispiel 5: Migräne

Seit Jahren ca. 6 Migränetage / Monat. Übelkeit, teilweise auch Erbrechen. Schmerzausstrahlung Hinterkopf bis Schläfe rechts. Pulsierender Schmerz. Erste Behandlung während einer Kopfschmerzphase. Platzierung von 3 Nadeln 0,25 x 40 Hinterkopf und 4 Nadeln 0,2 x 20 im Schmerzbereich Stirn und Schläfe, ohne Konstanthaltung der Stromstärke der einzelnen Nadeln. Nach der Behandlung Schmerzverstärkung insbesondere im Hinterkopf. Zweite Behandlung mit Konstanthaltung des Stroms jeder einzelnen Nadel bei 20 µA. Währen der Behandlung Abnahme der Übelkeit. Zwei Stunden nach der Behandlung Sistieren des Kopfschmerzes. In den darauf folgenden 8 Wochen statt der erwarteten 12 Migränetage nur zwei Migränetage. Erneute Behandlung in identischer Weise. Weitere Besserung auf einen Kopfschmerztag / Monat, jedoch ohne Übelkeit und Erbrechen.

## Patentansprüche

1. Gleichstromabgabevorrichtung umfassend eine Gleichstromquelle oder eine Einrichtung zum Anschließen an eine Gleichstromquelle und eine erste Elektrode sowie eine zweite Elektrode zum Verbinden mit der Gleichstromquelle, wobei die erste Elektrode als Mehrzahl von Nadeln ausgebildet ist und die zweite Elektrode als flächige Elektrode, als Nadel oder Mehrzahl unmittelbar miteinander elektrisch leitend verbundener Nadeln ausgebildet ist, **dadurch gekennzeichnet, dass** die Gleichstromabgabevorrichtung ein oder mehrere Mittel zum jeweiligen Konstanthalten der Einzelstromstärken des von individuellen Nadeln abgegebenen Gleichstroms bei der individuellen Abgabe von Gleichstrom durch individuelle Nadeln der ersten Elektrode umfasst, wobei die Nadeln der ersten Elektrode nicht unmittelbar miteinander elektrisch leitend verbunden sind.

2. Gleichstromabgabevorrichtung nach einem der vorherigen Ansprüche, wobei die zweite Elektrode als flächige Elektrode ausgebildet ist.

3. Gleichstromabgabevorrichtung nach einem der vorherigen Ansprüche, wobei das Konstanthalten ein automatisiertes Konstanthalten ist.

4. Gleichstromabgabevorrichtung nach einem der vorherigen Ansprüche, umfassend mehrere Mittel zum jeweiligen Konstanthalten der Stromstärke bei der individuellen Abgabe von Gleichstrom durch Nadeln der ersten Elektrode, wobei die Mittel Vorwiderstände sind.

5. Gleichstromabgabevorrichtung nach einem der vorherigen Ansprüche, wobei die jeweilige Stromstärke bei der individuellen Abgabe von Gleichstrom durch Nadeln der ersten Elektrode individuell einstellbar ist.

6. Gleichstromabgabevorrichtung nach einem der vorherigen Ansprüche, wobei die Spitzen der Mehrzahl von Nadeln der ersten und/oder der zweiten Elektrode entlang eines im Wesentlichen kreisförmigen oder elliptischen Umfangs angeordnet sind und wobei eine gegebenenfalls vorhandene unmittelbare elektrisch leitende Verbindung der Nadeln vorzugsweise entlang des Umfangs ausgebildet ist.

7. Gleichstromabgabevorrichtung nach einem der vorherigen Ansprüche, wobei die erste Elektrode als Kathode ausgebildet ist.

8. Gleichstromabgabevorrichtung nach einem Ansprüche 1 bis 7, wobei entweder (A) die zweite Elektrode als flächige Elektrode ausgebildet ist und vorzugsweise die maximale Gesamtstromstärke des Gleichstroms 2000 µA beträgt oder (B) die zweite Elektrode als Nadel ausgebildet ist und vorzugsweise die maximale Gesamtstromstärke des Gleichstroms 100 µA beträgt.

9. Verwendung einer Gleichstromabgabevorrichtung nach einem der Ansprüche 1 bis 7 zur kosmetischen Behandlung des menschlichen oder tierischen Körpers.

10. Verfahren zum Herstellen einer Gleichstromabgabevorrichtung nach einem der Ansprüche 1 bis 8, umfassend die folgenden Schritte: Bereitstellen eines Kits umfassend: eine Mehrzahl von Nadeln zur Verwendung als erste Elektrode, eine flächige Elektrode, eine Nadel oder eine Mehrzahl von Nadeln zur Verwendung als zweite Elektrode, ein oder mehrere Mittel zum jeweiligen Konstanthalten der Stromstärke bei der individuellen Abgabe von Gleichstrom durch mit einer Gleichstromquelle verbundene Nadeln der ersten Elektrode, und optional Mittel zum unmittelbaren elektrisch leitenden Verbinden einer Mehrzahl von Nadeln, Bereitstellen einer Gleichstromquelle, Bereitstellen der Mehrzahl von Nadeln zum Bilden der ersten Elektrode, Bereitstellen der flächigen Elektrode oder der Nadel oder unmittelbares elektrisch leitendes Verbinden der Mehrzahl von Nadeln zum Bilden der zweiten Elektrode, Verbinden der ersten Elektrode mit der Gleichstromquelle und Verbinden der zweiten Elektrode mit der Gleichstromquelle.

## Claims

1. Direct current output device comprising a direct source or a means for connecting to a direct current source, and a first electrode as well as a second electrode for connection with the direct current source, wherein the first electrode is designed as a plurality of needles and the second electrode as a spatial electrode, as a needle or a plurality of needles directly connected with each other in an electrically conductive way, **characterised in that** the direct current output device comprises one or more means for the respective constant holding of the individual current strengths of the direct currents emitted by individual needles during the individual output of direct current by individual needles of the first electrode, wherein the needles of the first electrode are not directly connected with each other in an electrically conductive way.

2. Direct current output device according to one of the preceding claims, wherein the second electrode is designed as a spatial electrode.

3. Direct current output device according to one of the preceding claims, wherein the constant holding is an automated constant holding.

4. Direct current output device according to one of the preceding claims, comprising several means for the respective constant holding of the current strength during the individual output of direct current by needles of the first electrode, wherein the means are series resistors.

5. Direct current output device according to one of the preceding claims, wherein the respective current strength can be set individually during the individual output of direct current by needles of the first electrode.

6. Direct current output device according to one of the preceding claims, wherein the tips of the plurality of needles of the first and/or the second electrode are arranged along a substantially circular or elliptical circumference, and wherein a possibly provided direct electrically conductive connection of the needles is preferably designed along the circumference.

7. Direct current output device according to one of the preceding claims, wherein the first electrode is designed as a cathode.

8. Direct current output device according to one of the claims 1 to 7, wherein either (A) the second electrode is designed as a spatial electrode and the maximum total current strength of the direct current is preferably 2000 µA, or (B) the second electrode is designed as a needle and the maximum total current strength is preferably 100 µA.

9. Use of a direct current output device according to claims 1 to 7 for the cosmetic treatment of the human or animal body.

10. Method for producing a direct current output device according to one of the claims 1 to 8, comprising the following steps: providing a kit, comprising: a plurality of needles for use as a first electrode, a spatial electrode, a needle of a plurality of needles for use as a second electrode, one or more means for the respective constant holding of the current strength during the individual output of direct current by needles connected with a direct current source of the first electrode, and optionally means for the direct connection of a plurality of needles in an electrically conductive way, providing a direct current source, providing the plurality of needles for forming the first electrode, providing the spatial electrode or the needle or the direct electrically conductive connecting of the plurality of needles for forming the second electrode, connecting the first electrode with the direct current source and connecting the second electrode with the direct current source.

## Revendications

1. Dispositif de fourniture de courant continu comprenant une source de courant continu ou un dispositif de raccordement à une source de courant continu et une première électrode ainsi qu'une deuxième électrode destinées à être reliées à la source de courant continu, la première électrode étant réalisée sous forme d'une pluralité d'aiguilles et la deuxième électrode étant réalisée sous forme d'une électrode plane, une aiguille ou une pluralité d'aiguilles reliées entre elles par une connexion électrique directe, **caractérisé par le fait que** le dispositif de fourniture de courant continu comprend un ou plusieurs moyens servant à maintenir constante l'intensité du courant continu délivré individuellement par les aiguilles de la première électrode, les aiguilles de la première électrode n'étant pas reliées entre elles par une connexion électrique directe.

2. Dispositif de fourniture de courant continu selon une des revendications précédentes, la deuxième électrode étant réalisée sous forme d'une électrode plane.

3. Dispositif de fourniture de courant continu selon une des revendications précédentes, l'intensité étant maintenue constante de manière automatique.

4. Dispositif de fourniture de courant continu selon une des revendications précédentes comprenant plusieurs moyens servant à maintenir constante l'intensité du courant continu délivré individuellement par les aiguilles de la première électrode, les moyens étant des résistances en série.

5. Dispositif de fourniture de courant continu selon une des revendications précédentes, chaque intensité de courant continu délivré individuellement par les aiguilles de la première électrode pouvant être réglée individuellement.

6. Dispositif de fourniture de courant continu selon une des revendications précédentes, les pointes de la pluralité d'aiguilles de la première et / ou de la deuxième électrode étant disposées le long d'une circonférence essentiellement circulaire ou elliptique et une connexion électrique directe éventuelle des aiguilles étant réalisée de préférence le long de la circonférence.

7. Dispositif de fourniture de courant continu selon une des revendications précédentes, la première électrode étant réalisée sous forme d'une cathode.

8. Dispositif de fourniture de courant continu selon une des revendications 1 à 7, soit (A) la deuxième électrode étant réalisée sous forme d'électrode plane et l'intensité maximale du courant continu total étant de préférence de 2 000 µA, soit (B) la deuxième électrode étant réalisée sous forme d'une aiguille et l'intensité maximale du courant continu total étant de préférence de 100 µA.

9. Utilisation d'un dispositif de fourniture de courant continu selon une des revendications 1 à 7 pour le traitement cosmétique du corps humain ou animal.

10. Procédé de fabrication d'un dispositif de fourniture de courant continu selon une des revendications 1 à 8 comprenant les étapes suivantes : fourniture d'un kit comprenant : une pluralité d'aiguilles pour être utilisées sous forme de première électrode, une électrode plane, une aiguille ou une pluralité d'aiguilles pour être utilisées comme deuxième électrode, un ou plusieurs moyens servant à maintenir constante l'intensité du courant continu délivré individuellement par les aiguilles de la première électrode reliées avec une source de courant continu et en option un moyen de connexion électrique directe d'une pluralité d'aiguilles ; fourniture d'une source de courant continu ; fourniture de la pluralité d'aiguilles pour réaliser la première électrode ; fourniture de l'électrode plane ou de l'aiguille ou connexion électrique directe de la pluralité d'aiguilles pour réaliser la deuxième électrode ; connexion de la première électrode avec la source de courant continu et connexion de la deuxième électrode avec la source de courant continu.
